# EUROPEAN PATENT SPECIFICATION

(11) **EP 4 061 337 B1**
(45) Date of publication and mention of the grant of the patent: **31.07.2024**
(21) Application number: 19845909.1
(22) Date of filing: 18.11.2019
(51) Int. Cl.: A61K 9/08, A61K 47/02, A61K 47/36, A61M 3/02

(54) **SOLUBLE PREPARATION FOR NASAL DOUCHES, CORRESPONDING NASAL IRRIGATION KIT AND OPERATING PROCEDURE FOR CARRYING OUT NASAL DOUCHES**
LÖSLICHE ZUBEREITUNG FÜR NASENDUSCHEN, ENTSPRECHENDES NASENSPÜLKIT UND BETRIEBSVERFAHREN ZUM DURCHFÜHREN VON NASENDUSCHEN
PRÉPARATION SOLUBLE POUR LAVAGE NASAL, TROUSSE D'IRRIGATION NASALE CORRESPONDANTE ET MODE OPÉRATOIRE POUR METTRE EN ?UVRE UN LAVAGE NASAL

(43) Date of publication of application: 28.09.2022
(73) Proprietor: Otosan S.r.l., 47122 Forli' (IT)
(72) Inventor: SETTANNI, Davide, 47121 FORLI' (IT)
(74) Representative: Modiano, Micaela Nadia
(86) International application number: PCT/IT2019/000105
(87) International publication number: WO 2021/100065

(56) References cited:
- WO-A2-2015/082965
- CN-A- 105 147 771
- CN-A- 108 743 612
- US-A1- 2002 158 089
- US-A1- 2013 039 950
- US-A1- 2014 199 266

## Description

The present invention relates to a soluble preparation for nasal douches, a corresponding nasal irrigation kit and an operating procedure for carrying out nasal douches.

The nose, over the course of the day, is constantly in contact with a great quantity of allergens, impurities and pollutant dust: usually the immune system of each individual protects them from the risks deriving from contact of these foreign substances with the nasal mucous membranes.

However, excessive exposure (for example owing to visiting environments that are particularly aggressive and/or polluted), a subjective predisposition (temporary or chronic), the effects of an illness and/or of an allergy, and other innumerable causes, can often appreciably increase the extent of the risks induced by these impurities.

Periodically carrying out nasal douches makes it possible to eliminate a large part of the impurities and foreign substances from the nasal mucous membranes, contributing to the reduction of the risks and reducing the negative effects induced by them (blocked nose, annoyance, dryness, irritation and, more generally, any alteration of the nasal mucous membrane).

The practice of nasal irrigation consists of making a saline solution in water flow inside the nose, making the stream of liquid enter one nostril and allowing it flow out of the other.

Numerous studies show that nasal irrigation, if performed daily, facilitates the cleaning of the mucous membrane, by favoring the expulsion of excess mucous, pollens, organic residues (skin, blood clots and the like) and other substances accumulated over the course of the day, which can compromise normal respiratory health.

Furthermore, the capacity to expel impurities and to keep the mucous membrane cleansed and hydrated makes nasal irrigation a complementary treatment for alleviating the symptoms of diseases of the upper respiratory pathways, accelerating recovery times and facilitating the restoration of the normal functions of the mucous membrane, being therefore particularly effective in the role of adjuvant in diseases like allergies, rhinitis, sinusitis, colds and the like.

The use is known of saline solutions (physiological saline) that are enriched, sometimes with some substances of proven beneficial effect (balsamic substances, disinfectants, softeners, cleansers) and assured compatibility with the organism of the users.

As an alternative, thermal spa waters are also marketed, suitably packaged.

Studies conducted on treatments with conventional washing solutions identify efficacy limitations that are common to all, and which to date have not been overcome.

The aim of the present invention is to solve the above mentioned drawbacks, by providing a soluble preparation for nasal douches that makes it possible to alleviate the symptoms of diseases of the upper respiratory pathways.

Within this aim, an object of the invention is to provide a soluble preparation for nasal douches that accelerates the recovery times from diseases of the upper respiratory pathways.

Another object of the invention is to provide a soluble preparation for nasal douches that facilitates the restoration of the normal functions of the mucous membrane, being effective in the role of adjuvant in diseases like allergies, rhinitis, sinusitis, colds and the like.

Another object of the invention is to provide a corresponding nasal irrigation kit that makes it possible to execute particularly efficacious nasal irrigation treatments directly in the home, by a user.

Another object of the invention is to provide an operating procedure for carrying out nasal douches that offers simple and immediate execution for any user.

Another object of the present invention is to provide a soluble preparation for nasal douches, a corresponding nasal irrigation kit and an operating procedure for carrying out nasal douches which are of low cost, easily and practically implemented, and safe in use.

This aim and these and other objects which will become better apparent hereinafter, are all achieved by a soluble preparation for nasal douches of the pulverulent type, of any granulometry, which comprises sodium chloride, characterized in that it comprises for every 1000 mg overall:
- sodium chloride in a quantity comprised between 710 mg, corresponding to 71% by weight, and 720 mg, corresponding to 72% by weight, in every 1000 mg of soluble preparation, as an agent for washing and breaking up bacterial film;
- sodium bicarbonate in a quantity comprised between 275 mg, corresponding to 27.5% by weight, and 290 mg, corresponding to 29% by weight, in every 1000 mg of soluble preparation , as a pH adjustment agent;
- hyaluronic acid in a quantity comprised between 2.9 mg, corresponding to 0.29% by weight, and 3.2 mg, corresponding to 0.32% by weight, in every 1000 mg of soluble preparation, as an agent of a type chosen from among softening, moisturizing, protective, inductive of mucociliary motility, tissue restructuring, and a combination thereof.

Such aim and such objects are also achieved by a nasal irrigation kit, characterized in that it comprises:
- at least one package containing between 2600 mg and 3000 mg of a soluble preparation according to claim 1;
- at least one nasal irrigation bottle, which has an inner cavity adapted to contain between 200 ml and 300 ml of a solution, and which is provided with at least one nozzle of shape and size substantially complementary to those of a human nostril.

Such aim and such objects are also achieved by an operating procedure for carrying out nasal douches, which consists of:
- pouring a predefined quantity of lukewarm water into a main body of a bottle,
- pouring a predefined quantity of soluble preparation according to claim 1 into said main body of said bottle;
- waiting for the particles of soluble preparation, or solute, to dissolve completely in the water, or solvent;
- closing said main body of said bottle with a respective cap which is provided with a nozzle of shape and size substantially complementary to those of a human nostril;
- fitting said nozzle into a nostril of a user and dispensing said solution so that it enters inside such nostril and flows through the nasal cavity, exiting from the other nostril.

Further characteristics and advantages of the invention will become better apparent from the detailed description that follows of a preferred, but not exclusive, embodiment of the soluble preparation for nasal douches, of the corresponding nasal irrigation kit, which are used via the operating procedure for carrying out nasal douches according to the invention, which is illustrated by way of non-limiting example in the accompanying drawings, wherein:

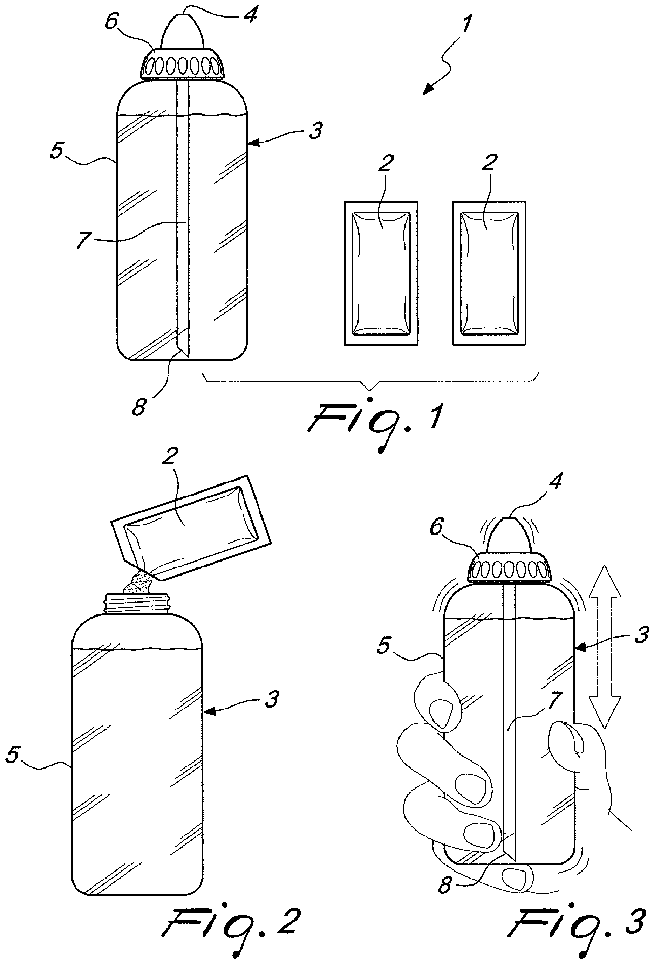

Figure 1 is a schematic front elevation view of a nasal irrigation kit according to the invention;
Figure 2 is a schematic front elevation view of the step of introducing the soluble preparation, contained in a specific package, into a bottle containing a liquid, the package and the bottle constituting the kit according to the invention;
Figure 3 is a schematic front elevation view of the step of mixing the soluble preparation with water inside the bottle belonging to the kit according to the invention;

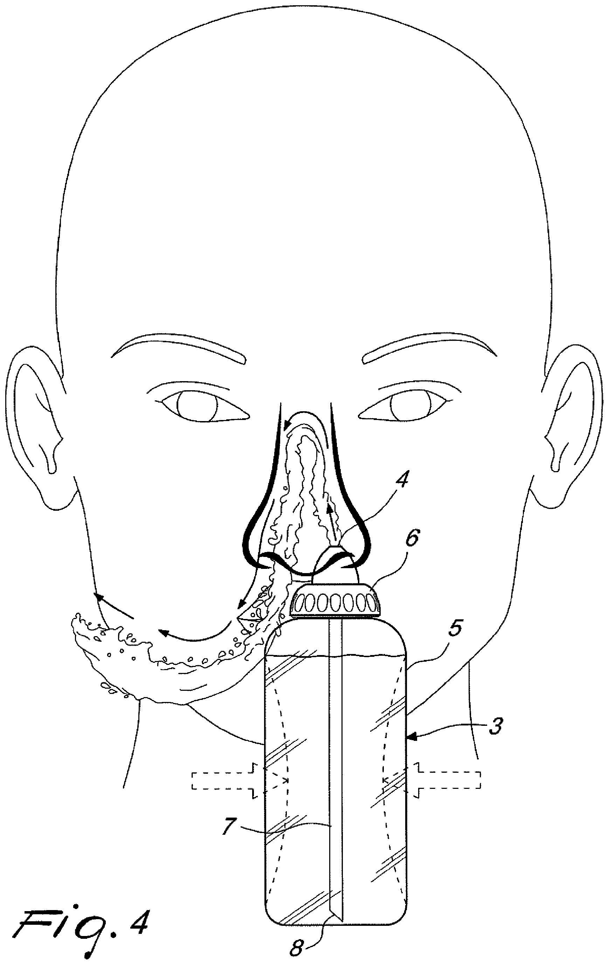

Figure 4 is a schematic front elevation view of the method of carrying out a nasal irrigation performed with a bottle belonging to the kit according to the invention.

With particular reference to the figures, the reference numeral 1 generally designates a nasal irrigation kit according to the invention.

The soluble preparation for nasal douches of the pulverulent type: dust in particular can have any granulometry and comprise sodium chloride.

The soluble preparation according to the invention, for each 1000 mg overall, comprises:
- a quantity of sodium chloride comprised between 710 mg, corresponding to 71% by weight, and 720 mg, corresponding to 72% by weight, in every 1000 mg of soluble preparation, as an agent for washing and breaking up bacterial film;
- a quantity of sodium bicarbonate comprised between 275 mg, corresponding to 27.5% by weight, and 290 mg, corresponding to 29% by weight, in every 1000 mg of soluble preparation, as a pH adjustment agent;
- a quantity of hyaluronic acid comprised between 2.9 mg, corresponding to 0.29% by weight, and 3.2 mg, corresponding to 0.32% by weight, in every 1000 mg of soluble preparation, as an agent of a type chosen from among softening, moisturizing, protective, inductive of mucociliary motility, tissue restructuring, and a combination thereof.

The soluble preparation according to the invention will preferably be supplied in doses made up of about 2600/3000 mg: in particular the provision is envisaged of individual packages 2 of soluble preparation which contain about 2838/2840 mg of preparation. Nasal irrigation treatments implemented using the soluble preparation according to the invention can conveniently entail the use of a single package 2 (which corresponds to about 2838/2840 mg of preparation) or of two packages 2 simultaneously. Any simultaneous uses of a number of packages 2 exceeding two, although envisaged, can preferably be carried out only after suitable monitoring by a doctor.

In particular it should be noted that an excellent effect of washing and breaking up bacterial film has been found by using a quantity of approximately 715 mg of sodium chloride for each 1000 mg of soluble preparation.

With particular reference to the single package 2 described above (containing about 2838/2840 mg of soluble preparation), it should be noted that this will contain about 2029/2030 mg of sodium chloride.

It should furthermore be noted that the sodium chloride used will not comprise significant traces of iodates, iodides or other derivatives of iodine, as this element brings no advantage to the present application and, on the contrary, it may, in some cases, induce unwanted effects.

In particular the capacity has been found of generating an optimal regulation of the pH overall (bringing it indicatively to values comprised between 7.2 and 8.3, which are the bodily values of the nasal mucous membrane during rhinitis) by using a quantity of approximately 282 mg of sodium bicarbonate for each 1000 mg of soluble preparation.

With particular reference to the single package 2 described above (containing about 2838/2840 mg of soluble preparation), it should be noted that this will contain preferably about 800/801 mg of sodium bicarbonate.

In detail, specific tests and medical studies have confirmed evident softening, moisturizing and protective effects, combined with the induction of optimal mucociliary motility and an excellent restructuring of the tissues, if a quantity comprised in the range between 3 mg and 3.1 mg of hyaluronic acid is used for each 1000 mg of soluble preparation.

With particular reference to the single package 2 described above (containing about 2838/2840 mg of soluble preparation), it should be noted that this will contain preferably about 9 mg of hyaluronic acid.

The present invention also extends its protection to the nasal irrigation kit 2, which is constituted by at least one package containing between 2600 mg and 3000 mg of the soluble preparation described previously, by at least one nasal irrigation bottle 3, which has an inner cavity that is adapted to contain between 200 ml and 300 ml of a solution.

The bottle 3 is furthermore provided with at least one nozzle 4 of shape and size substantially complementary to those of a human nostril: during use, the nozzle 4 must be juxtaposed with the nostril of the user in order to ensure that introduction of the solution into the nasal duct takes place while limiting (or completely eliminating) any dispersion of the solution itself.

With particular reference to an embodiment of undoubted practical interest, it should be noted that the bottle 3 can conveniently comprise a main body 5 made of elastically deformable material, which is provided with an upper filling opening which can be coupled hermetically to a closing cap 6 which is provided with a projection which defines the nozzle 4.

It should furthermore be noted that the nozzle 4 is coupled hermetically to a tube 7 of length substantially similar to the depth of the inner cavity of the main body 5.

In the configuration in which the cap 6 is coupled to the main body 5 (which can be obtained by virtue of an elastic connection and/or a threaded connection and/or other conventional connection methods), the end face 8 of the tube 7 will be faced toward and proximate to the bottom of the inner cavity of the main body 5.

When the main body 5 is squeezed by a user (who squeezes directly with the hand with which the user holds the bottle 3), when the cap 6 is hermetically closed, the liquid contained in the main body 5 will flow into the tube 7 and subsequently flow outward through the nozzle 4. By keeping the nozzle 4 in juxtaposition with a nostril of the user, the irrigation of the nasal cavity of the user will be achieved with the solution contained in the bottle 3.

Profitably the main body 5 can be graduated, so as to indicate to the user the volume of material contained in it: the term "material" is used to mean any constituent material selected from water (or another solvent, in specific applications), soluble preparation, and a solution thereof.

The graduated scale present on the main body 5 allows the user to divide the treatment, irrigating first one nostril with a first part of the solution contained in the bottle 4 and then the second nostril with the remaining solution.

The protection offered by the present invention also refers to an operating procedure for carrying out nasal douches which involves the execution of a predefined sequence of steps.

Firstly it is necessary to pour a predefined quantity of lukewarm water into the main body 5 of the bottle 3.

In a subsequent step, it will be necessary to pour a predefined quantity of the soluble preparation described previously into the main body 5 of the bottle 3.

Then the user waits for the particles of soluble preparation, or solute, to dissolve completely in the water that acts as a solvent.

In a subsequent step, it will then be possible to close the main body 5 of the bottle 3 with the respective cap 6 which is provided with the nozzle 4 of shape and size substantially complementary to those of a human nostril.

To carry out the douche, the nozzle 4 must then be fitted into a nostril of a user and the solution dispensed so that it enters inside such nostril and flows through the nasal cavity, exiting from the other nostril.

It should be noted that, in the method just described, the water introduced into the main body 5 should preferably correspond to a volume comprised between 230 ml and 250 ml and have a temperature comprised between 30° C and 45° C.

An embodiment of the process according to the invention which is of proven efficacy entails the introduction of 240 ml of water at a temperature of between 35°C and 40°C in the bottle 3.

Attention is again drawn to the simplicity with which the operations to actually wash the nasal ducts can be carried out, and this simplicity is ensured by the delivery of the solution produced by the squeezing, by the user, of the main body 5 which is closed hermetically by the cap 6.

The squeezing of the main body 5 in fact causes the entrance of the solution (contained in it) into a tube 7, which has an end face facing toward and proximate to the bottom of the main body 5 and the opposite end hermetically coupled to the cap 6 and leading to the nozzle 4, and its flow outward through that nozzle 4. The force with which the user exerts the pressure on the main body 5 (by squeezing it) will also determine the exit speed of the solution from the nozzle 4, which, in turn, can contribute to a mechanical ablation of the foreign agents present in the nasal duct.

The dispensing of the solution is preferably and advantageously carried out, for a first quantity (generally constituted by half of the overall quantity of solution present in the bottle 3), by fitting the nozzle 4 into a first nostril of the user, with consequent expulsion of the solution from a second nostril (after the solution has flowed inside the nasal cavities of the user), and, for a second quantity (the part of solution remaining inside the bottle), by fitting the nozzle into the second nostril of the user, with consequent expulsion of the solution from the first nostril (after the solution has flowed inside the nasal cavities of the user).

The present invention makes it possible to combine the old method of nasal douches with the more modern requirements of practicality, by virtue of the adoption of a nasal irrigation kit 1, which is easy and quick to use.

The commercialization of specifically and precisely dosed packages 2, associated with a specific irrigation bottle 3, specially designed to ensure an optimal pressure of the flow dispensed, enables each user (including users who have no previous experience with nasal irrigation operations) to properly and successfully carry out all the necessary operations for the daily hygiene of the nasal mucous membranes.

Such operations promote the process of cleaning the nasal mucous membrane, favor mucociliary transport, improve breathing, and combat the stagnation of secretions and the consequent process of contamination.

The present invention is therefore particularly useful for treating nasal congestion, colds or allergic rhinitis, acute and chronic sinusitis, hypertrophic rhinitis in pregnancy, snoring, time spent in dry or polluted environments, exposure to smoke or irritants, pre- and post-operating nasal hygiene, and daily cleaning of the nasal passages.

It should be noted that tests have been carried out to administer, using the kit 1, the solution obtained with the soluble preparation according to the invention on users affected by rhino-sinusitis and on users who in the past have been subjected to surgery on the nasal ducts.

Such conditions further determine the swelling of the nasal mucous membrane, which can be associated with pain, stinging and annoyance to a varied extent.

After carrying out repeated treatments (carried out multiple times per day), which must be extended for a number of days indicated by the doctor in charge of the test (repeating the patient control examinations with a predefined regularity, for example after 5, 15 and 30 days) a considerable reduction was found in swelling of the mucous membranes and in the corresponding pain and stinging.

Furthermore a clear reduction was observed in the bleeding of surgical wounds, together with their good healing.

Overall, all the users subjected to the test improved their conditions, including compared to users who carried out equivalent irrigation operations with conventional solutions. In particular, after 30 days the results obtained were shown to be particularly evident and shared by all users who were subjected to the test.

The capacity of the solution obtained by virtue of using the soluble preparation according to the invention has therefore been shown to be particularly suitable for favoring the optimal cleaning of the nasal cavities, removing excess secretions and stimulating the restoration of mucociliary transport. The bottle 3 present in the kit 1 according to the invention further makes it possible to modulate the pressure of the jet, so enabling an efficacious breaking-up of the bio-bacterial film present on the mucous membranes.

The properties conferred by hyaluronic acid on the solution that comprises the soluble preparation determine (by virtue of the specific dose selected to maximize the positive effects) an intense synergistic effect with the other constituents (i.e. sodium chloride and sodium bicarbonate in the specific doses described above), favoring mucociliary drainage and the re-epithelialization of the nasal mucous membrane.

Moreover, it should be noted that the solution obtained with the soluble preparation according to the invention does not produce any side effect, even in combination with medicines administered to the user.

Advantageously the present invention solves the above mentioned problems, by providing a soluble preparation for nasal douches that makes it possible to alleviate the symptoms of diseases of the upper respiratory pathways.

Conveniently the soluble preparation for nasal douches according to the invention accelerates the recovery times from diseases of the upper respiratory pathways.

Positively the soluble preparation for nasal douches according to the invention facilitates the restoration of the normal functions of the mucous membrane, being effective in the role of adjuvant in diseases like allergies, rhinitis, sinusitis, colds and the like.

Usefully, the nasal irrigation kit according to the invention makes it possible to execute particularly efficacious nasal irrigation treatments directly in the home, by a user.

Advantageously the operating procedure for carrying out nasal douches according to the invention offers simple and immediate execution for any user.

Positively the soluble preparation for nasal douches and the corresponding nasal irrigation kit 1 are easily and practically implemented and are of low cost, also allowing the adoption of an operating procedure for carrying out nasal douches that is simple and within the abilities of any user: such characteristics ensure that the soluble preparation, the irrigation kit 1 and also the method for use according to the invention are therefore innovations that are certain to be safe in use.

The invention, thus conceived, is susceptible of numerous modifications and variations, all of which are within the scope of the appended claims. Moreover, all the details may be substituted by other, technically equivalent elements.

In the embodiments illustrated, individual characteristics shown in relation to specific examples may in reality be interchanged with other, different characteristics, existing in other embodiments.

In practice, the materials employed, as well as the dimensions, may be any according to requirements and to the state of the art.

Where the technical features mentioned in any claim are followed by reference numerals and/or signs, those reference numerals and/or signs have been included for the sole purpose of increasing the intelligibility of the claims and accordingly, such reference numerals and/or signs do not have any limiting effect on the interpretation of each element identified by way of example by such reference numerals and/or signs.

## Claims

1. A soluble preparation for nasal douches of the pulverulent type, of any granulometry, which comprises sodium chloride, **characterized in that** it comprises for every 1000 mg overall:
- sodium chloride in a quantity comprised between 710 mg, corresponding to 71% by weight, and 720 mg, corresponding to 72% by weight, in every 1000 mg of soluble preparation , as an agent for washing and breaking up bacterial film;
- sodium bicarbonate in a quantity comprised between 275 mg, corresponding to 27.5% by weight, and 290 mg, corresponding to 29% by weight, in every 1000 mg of soluble preparation , as a pH adjustment agent;
- hyaluronic acid in a quantity comprised between 2.9 mg, corresponding to 0.29% by weight, and 3.2 mg, corresponding to 0.32% by weight, in every 1000 mg of soluble preparation, as an agent of a type chosen from among softening, moisturizing, protective, inductive of mucociliary motility, tissue restructuring, and a combination, thereof.

2. A nasal irrigation kit, **characterized in that** it comprises:
- at least one package (2) containing between 2600 mg and 3000 mg of a soluble preparation according to claim 1;
- at least one nasal irrigation bottle (3), which has an inner cavity adapted to contain between 200 ml and 300 ml of a solution, and which is provided with at least one nozzle (4) of shape and size substantially complementary to those of a human nostril.

3. The kit according to claim 2, **characterized in that** said bottle (3) comprises a main body (5) made of elastically deformable material, which is provided with an upper filling opening which can be coupled hermetically to a closing cap (6) which is provided with a projection which defines said nozzle (4).

4. The kit according to claim 5, **characterized in that** said nozzle (4) is hermetically coupled to a tube (7) of length substantially similar to the depth of the inner cavity of said main body (5), in the configuration in which said cap (6) is coupled to said main body (5) the end face (8) of said tube (7) being faced toward and proximate to the bottom of the inner cavity of said main body (5), a squeezing of said main body (5) by a user, when said cap (6) is hermetically closed, determining the entrance of the liquid, initially contained in the main body (5), into said tube (7) and its flow outward through said nozzle (4).

5. The kit according to claim 2, **characterized in that** said main body (5) is graduated in order to indicate the volume of material contained in it, said material being constituted by an element selected from water, said soluble preparation, and a solution thereof.

6. A soluble preparation for use in an operating procedure for carrying out nasal douches, which consists of:
- pouring a predefined quantity of lukewarm water into a main body (5) of a bottle (3),
- pouring a predefined quantity of soluble preparation according to claim 1 into said main body (5) of said bottle (3);
- waiting for the particles of soluble preparation, or solute, to dissolve completely in the water, or solvent;
- closing said main body (5) of said bottle (3) with a respective cap (6) which is provided with a nozzle (4) of shape and size substantially complementary to those of a human nostril;
- fitting said nozzle (4) into a nostril of a user and dispensing said solution so that it enters inside such nostril and flows through the nasal cavity, exiting from the other nostril.

7. The operating procedure according to claim 6, **characterized in that** the water introduced into said main body has a volume comprised between 230 ml and 250 ml and has a temperature comprised between 30° C and 45° C.

8. The operating procedure according to claim 6, **characterized in that** said dispensing of said solution is caused by the squeezing, by said user, of said main body (5) which is closed hermetically by said cap (6), with the consequent entrance of the solution contained in it into a tube (7), which has an end face (8) facing toward and proximate to the bottom of said main body (5) and the opposite end hermetically coupled to said cap (6) and leading to said nozzle (4), and its flow outward through said nozzle (4).

9. The operating procedure according to claim 6, **characterized in that** said dispensing of said solution is carried out, for a first quantity, by fitting said nozzle (4) into a first nostril of said user with consequent expulsion of the solution from a second nostril, and, for a second quantity, by fitting said nozzle (4) into said second nostril of said user with consequent expulsion of the solution from said first nostril.

## Patentansprüche

1. Eine lösliche Zubereitung für Nasenduschen vom pulverförmigen Typ mit beliebiger Granulometrie, die Natriumchlorid umfasst; **dadurch gekennzeichnet, dass** sie für jeweils 1000 mg insgesamt Folgendes umfasst:
- Natriumchlorid in einer Menge zwischen 710 mg, entsprechend 71 Gewichtsprozent, und 720 mg, entsprechend 72 Gewichtsprozent, in jeweils 1000 mg löslicher Zubereitung, als Mittel zum Spülen und Auflösen von bakteriellem Film;
- Natriumbicarbonat in einer Menge zwischen 275 mg, entsprechend 27,5 Gewichtsprozent, und 290 mg, entsprechend 29 Gewichtsprozent, in jeweils 1000 mg löslicher Zubereitung, als Mittel zur Einstellung des pH-Werts;
- Hyaluronsäure in einer Menge zwischen 2,9 mg, entsprechend 0,29 Gewichtsprozent, und 3,2 mg, entsprechend 0,32 Gewichtsprozent, in jeweils 1000 mg löslicher Zubereitung, als Mittel von einer Art gewählt aus Erweichungs-, Befeuchtungs-, Schutzmitteln, Mitteln zur Förderung der mukoziliaren Motilität, Gewebe-Neustrukturierung und einer Kombination davon.

2. Ein Nasenspülungskit, **dadurch gekennzeichnet, dass** es Folgendes umfasst:
- mindestens eine Verpackung (2), die zwischen 2600 mg und 3000 mg einer löslichen Zubereitung gemäß Anspruch 1 enthält;
- mindestens eine Nasenspülungsflasche (3), die einen inneren Hohlraum hat, ausgebildet, um zwischen 200 ml und 300ml einer Lösung aufzunehmen, und die mit mindestens einer Düse (4) mit einer Form und Größe ausgestattet ist, welche im Wesentlichen komplementär zu denjenigen eines menschlichen Nasenlochs sind.

3. Das Kit gemäß Anspruch 2, **dadurch gekennzeichnet, dass** die Flasche (3) einen Hauptkörper (5) aus elastisch verformbarem Material umfasst, der mit einer oberen Füllöffnung ausgestattet ist, die hermetisch mit einer Verschlusskappe (6) verbunden werden kann, welche mit einem Vorsprung versehen ist, der die Düse (4) bestimmt.

4. Das Kit gemäß Anspruch 5, **dadurch gekennzeichnet, dass** die Düse (4) hermetisch mit einer Röhre (7) von einer Länge verbunden ist, die im Wesentlichen ähnlich der Tiefe des inneren Hohlraums des Hauptkörpers (5) ist, wobei in der Anordnung, in welcher die Kappe (6) mit dem Hauptkörper (5) gekoppelt ist, die Endfläche (8) der Röhre (7) dem Boden des inneren Hohlraums des Hauptkörpers (5) zugewandt ist und an ihn angrenzt; wobei ein Drücken des Hauptkörpers (5) durch einen Benutzer, wenn die Kappe (6) hermetisch geschlossen ist, den Eintritt der Flüssigkeit, die ursprünglich im Hauptkörper (5) enthalten ist, in die Röhre (7) und ihr Fließen nach außen durch die Düse (4) bestimmt.

5. Das Kit gemäß Anspruch 2, **dadurch gekennzeichnet, dass** der Hauptkörper (5) mit einer Einteilung versehen ist, um das Volumen des in ihm enthaltenen Materials anzuzeigen, wobei das Material aus einem Element besteht, das gewählt ist aus Wasser, der löslichen Zubereitung und einer Lösung davon.

6. Eine lösliche Zubereitung zur Verwendung in einem Arbeitsverfahren für die Durchführung von Nasenduschen, das in Folgendem besteht:
- Gießen einer vordefinierten Menge lauwarmen Wassers in einen Hauptkörper (5) einer Flasche (3),
- Gießen einer vordefinierten Menge löslicher Zubereitung gemäß Anspruch 1 in den Hauptkörper (5) der Flasche (3),
- Warten darauf, dass sich die Partikel der löslichen Zubereitung, oder des gelösten Stoffs, im Wasser, oder Lösungsmittel, vollständig auflösen;
- Schließen des Hauptkörpers (5) der Flasche (3) mit einer entsprechenden Kappe (6), die mit einer Düse (4) von einer Form und Größe ausgestattet ist, die im Wesentlichen komplementär zu denjenigen eines menschlichen Nasenlochs sind;
- Einführen der Düse (4) in ein Nasenloch eines Benutzers und Abgeben der Lösung, so dass sie in ein solches Nasenloch eintritt, durch die Nasenhöhle strömt und aus dem anderen Nasenloch austritt.

7. Das Arbeitsverfahren gemäß Anspruch 6, **dadurch gekennzeichnet, dass** das in den Hauptkörper eingefüllte Wasser ein Volumen zwischen 230 ml und 250 ml und eine Temperatur zwischen 30°C und 45°C hat.

8. Das Arbeitsverfahren gemäß Anspruch 6, **dadurch gekennzeichnet, dass** das Abgeben der Lösung durch das Drücken des Hauptkörpers (5), der hermetisch durch die Kappe (6) verschlossen ist, durch den Benutzer veranlasst wird, mit daraus folgendem Eintritt der in ihm enthaltenen Lösung in eine Röhre (7), die eine Endfläche (8) hat, welche dem Boden des Hauptkörpers (5) zugewandt ist und an ihn angrenzt, und das gegenüberliegende Ende hermetisch mit der Kappe (6) verbunden ist und zu der Düse (4) führt; und ihr Fließen durch die Düse (4) nach außen.

9. Das Arbeitsverfahren gemäß Anspruch 6, **dadurch gekennzeichnet, dass** das Abgeben der Lösung für eine erste Menge durchgeführt wird durch Einführen der Düse (4) in ein erstes Nasenloch des Benutzers mit daraus folgendem Ausstoßen der Lösung aus einem zweiten Nasenloch; und für eine zweite Menge durch Einführen der Düse (4) in das zweite Nasenloch des Benutzers mit daraus folgendem Ausstoßen der Lösung aus dem ersten Nasenloch.

## Revendications

1. Préparation soluble pour douches nasales du type pulvérulent, de toute granulométrie, qui comprend du chlorure de sodium, **caractérisée en ce qu'**elle comprend, pour chaque 1000 mg au total :
- du chlorure de sodium en une quantité comprise entre 710 mg, correspondant à 71 % en poids, et 720 mg, correspondant à 72 % en poids, dans chaque 1000 mg de préparation soluble, en tant qu'agent de lavage et de rupture du film bactérien ;
- du bicarbonate de sodium en une quantité comprise entre 275 mg, correspondant à 27,5 % en poids, et 290 mg, correspondant à 29 % en poids, dans chaque 1000 mg de préparation soluble en tant qu'agent d'ajustement du pH ;
- de l'acide hyaluronique en une quantité comprise entre 2,9 mg, correspondant à 0,29 % en poids, et 3,2 mg, correspondant à 0,32 % en poids, dans chaque 1000 mg de préparation soluble, en tant qu'agent d'un type choisi parmi un adoucissant, un hydratant, un protecteur, un inducteur de la motilité mucociliaire, de la restructuration des tissus, et une combinaison de ceux-ci.

2. Kit d'irrigation nasale, **caractérisé en ce qu'**il comprend :
- au moins un emballage (2) contenant entre 2600 mg et 3000 mg d'une préparation soluble selon la revendication 1 ;
- au moins un flacon d'irrigation nasale (3), qui comporte une cavité interne adaptée pour contenir entre 200 ml et 300 ml d'une solution, et qui est pourvu d'au moins une buse (4) de forme et de taille sensiblement complémentaires de celles d'une narine humaine.

3. Kit selon la revendication 2, **caractérisé en ce que** ledit flacon (3) comprend un corps principal (5) constitué d'un matériau élastiquement déformable, qui est pourvu d'une ouverture de remplissage supérieure qui peut être couplée hermétiquement à un capuchon de fermeture (6) qui est pourvu d'une saillie qui définit ladite buse (4).

4. Kit selon la revendication 5, **caractérisé en ce que** ladite buse (4) est couplée hermétiquement à un tube (7) de longueur sensiblement similaire à la profondeur de la cavité interne dudit corps principal (5), dans la configuration dans laquelle ledit capuchon (6) est couplé audit corps principal (5), la face d'extrémité (8) dudit tube (7) étant tournée vers et à proximité du fond de la cavité interne dudit corps principal (5), une compression dudit corps principal (5) par un utilisateur, lorsque ledit capuchon (6) est hermétiquement fermé, déterminant l'entrée du liquide, initialement contenu dans le corps principal (5), dans ledit tube (7) et son écoulement vers l'extérieur à travers ladite buse (4).

5. Kit selon la revendication 2, **caractérisé en ce que** ledit corps principal (5) est gradué pour indiquer le volume de matière qu'il contient, ladite matière étant constituée par un élément choisi parmi l'eau, ladite préparation soluble et une solution de celle-ci.

6. Préparation soluble destinée à être utilisée dans une procédure opératoire pour effectuer des douches nasales, qui consiste à :
- verser une quantité prédéfinie d'eau tiède dans un corps principal (5) d'un flacon (3),
- verser une quantité prédéfinie de préparation soluble selon la revendication 1 dans ledit corps principal (5) dudit flacon (3) ;
- attendre que les particules de préparation soluble, ou de soluté, se dissolvent complètement dans l'eau, ou le solvant ;
- fermer ledit corps principal (5) dudit flacon (3) avec un capuchon respectif (6) qui est pourvu d'une buse (4) de forme et de taille sensiblement complémentaires de celles d'une narine humaine ;
- insérer ladite buse (4) dans une narine d'un utilisateur et distribuer ladite solution de sorte qu'elle pénètre à l'intérieur de ladite narine et s'écoule à travers la cavité nasale, en sortant par l'autre narine.

7. Procédure opératoire selon la revendication 6, **caractérisée en ce que** l'eau introduite dans ledit corps principal a un volume compris entre 230 ml et 250 ml et a une température comprise entre 30 °C et 45 °C.

8. Procédure opératoire selon la revendication 6, **caractérisée en ce que** ladite distribution de ladite solution est provoquée par la compression, par ledit utilisateur, dudit corps principal (5) qui est fermé hermétiquement par ledit capuchon (6), entraînant l'entrée de la solution contenue dans celui-ci dans un tube (7), qui a une face d'extrémité (8) orientée vers le fond dudit corps principal (5) et à proximité de celui-ci, et l'extrémité opposée hermétiquement couplée audit capuchon (6) et menant à ladite buse (4), et son écoulement vers l'extérieur à travers ladite buse (4).

9. Procédure opératoire selon la revendication 6, **caractérisée en ce que** ladite distribution de ladite solution est réalisée, pour une première quantité, par ajustement de ladite buse (4) dans une première narine dudit utilisateur entraînant l'expulsion de la solution depuis une deuxième narine, et, pour une deuxième quantité, par ajustement de ladite buse (4) dans ladite deuxième narine dudit utilisateur entraînant l'expulsion de la solution depuis ladite première narine.
